Europäisches Patentamt

European Patent Office

Office européen des brevets

(11) Veröffentlichungsnummer : **0 430 975 B1**

## EUROPÄISCHE PATENTSCHRIFT

(45) Veröffentlichungstag der Patentschrift :
**23.12.92 Patentblatt 92/52**

(51) Int. Cl.⁵ : **G01B 7/06,** G01B 7/28, G01B 21/08

(21) Anmeldenummer : **89908816.5**

(22) Anmeldetag : **01.08.89**

(86) Internationale Anmeldenummer :
**PCT/DE89/00499**

(87) Internationale Veröffentlichungsnummer :
**WO 90/01673 22.02.90 Gazette 90/05**

(54) **MESSVORRICHTUNG ZUR QUERPROFILMESSUNG EINER PAPIERBAHN.**

(30) Priorität : **10.08.88 DE 3827084**

(43) Veröffentlichungstag der Anmeldung :
**12.06.91 Patentblatt 91/24**

(45) Bekanntmachung des Hinweises auf die
Patenterteilung :
**23.12.92 Patentblatt 92/52**

(84) Benannte Vertragsstaaten :
**AT FR GB SE**

(56) Entgegenhaltungen :
**DE-A- 2 005 655**
**GB-A- 1 228 001**

(56) Entgegenhaltungen :
US-A- 3 609 318
US-A- 4 271 699
**Patent Abstracts of Japan, Vol. 10, Nr. 26
(P-425)(2083), 31 January 1986 & JP-
A-60177202**

(73) Patentinhaber : **SULZER-ESCHER WYSS
GMBH
Escher Wyss-Strasse 25 Postfach 1380
W-7980 Ravensburg (DE)**

(72) Erfinder : **MÜNCH, Rudolf
Mittelöschstr. 3
W-7980 Ravensburg (DE)**
Erfinder : **WEISSHUHN, Elmer
Tilsiterstr. 29
W-7981 Vogt (DE)**

EP 0 430 975 B1

Jouve, 18, rue Saint-Denis, 75001 PARIS

**Beschreibung**

Die Erfindung betrifft eine Messvorrichtung gemäß dem Oberbegriff des Patentanspruchs 1.

Es ist bis heute üblich, den Messkopf mit einer immer gleichen Traversiergeschwindigkeit der Messbrücke entlang, quer zur Papierbahn, zu bewegen. Dies führt dazu, daß die Messungen an den einzelnen Messorten in einem immer gleichen Zeitraster erfolgen.

Ziel der Messung ist, das stabile Querprofil der Bahn zu ermitteln und demnach Korrekturen am Stoffauflauf, z.B. durch notwendige Einstellung der lichten Weiten des Stoffauflaufspaltes - längs und quer zur Laufrichtung - vorzunehmen, um ein gewünschtes Querprofil der Bahn zu erzielen. Die Bildung des gewünschten Querprofils der Bahn wird durch Störungen im Stoffstrom aus dem Stoffauflauf beeinträchtigt. Diese Störungen, die Abweichungen von dem gewünschten Querprofi> zur Folge haben, können nicht immer ganz präzise erfaßt werden. Es kommt vor, daß die Frequenz der Störung und die Frequenz der Messung in einem ganzzahligen Verhältnis stehen. Dann kann das Messergebnis eine Abweichung vom gewünschten Querprofil vortäuschen. Eine Korrektur nach solcher Messung kann daher nicht zum Erzielen des gewünschten Querprofils der Bahn führen.

Die GB-A-1 228 001 zeigt eine Meßvorrichtung zur Ermittlung von Durchschnittswerten z. B. für die Dicke einer vorbeilaufenden Bahn. Dabei wird die Abtastgeschwindigkeit proportional zur Bahnlaufgeschwindigkeit geregelt. Dieses Meßsystem vermag allerdings nicht, die aus der Übereinstimmung von Meßfrequenz und Störfrequenz herrührende Verfälschung des Ergebnisses zu verhindern.

Es ist Aufgabe der Erfindung, eine Vorrichtung, bzw. ein Verfahren zur Messung des Querprofils der Papierbahn vorzuschlagen zur Ermittlung des stabilen Querprofils über eine betrachtete Länge der Papierbahn. Dies soll Fehlbeurteilungen des Querprofils und in der Folge unzweckmäßige Stelleingriffe an den das Querprofil der Papierbahn beeinflussenden Stellvorrichtungen verhindern.

Diese Aufgabe ist an gattungsgleichen Vorrichtungen durch Maßnahmen, die im Patentanspruch 1 angegeben sind, erfüllt.

Im Anspruch 2 ist eine vorteilhafte Ausführung des Erfindungsgegenstandes angegeben und im Anspruch 3 eine sinnvolle Anwendung.

Im weiteren wird der Erfindungsgegenstand näher beschrieben und erklärt. Die Beschreibung bezieht sich auf eine Zeichnung, in welcher zeigt:

Fig. 1 eine schematische Darstellung des Erfindungsgegenstandes in Draufsicht.

Fig. 2 eine Darstellung eines Messverfahrens mit der erfindungsgemäßen Vorrichtung.

Die Messvorrichtung 1 zur Querprofilmessung einer Materialbahn, hier einer Papierbahn 2, die nach einem Stoffauflauf 3 gebildet wird und sich in Richtung des eingezeichneten Pfeiles mit einer bestimmten Geschwindigkeit bewegt, weist eine Messbrücke 4 auf, welche ortsfest über der Bahn 2 und quer zu ihr angeordnet ist. Auf der Messbrücke 4 befindet sich ein Messkopf 5, welcher entlang der Messbrücke 4, d.h. quer zu der unter der Messbrücke sich bewegenden Bahn 2 hin und her bewegbar ist:. Diese Bewegung wird erzeugt durch einen reversiblen und drehzahlregelbaren Antriebsmotor 6. Geregelt wird der Motor 6 von einem Prozeß-Leitsystem 7, dem auch die jeweils an den Messmarken 12 gemessenen Messwerte zur Beurteilung des Querprofils zugeführt werden.

Die einzelnen Messungen erfolgen jeweils entlang gedachter Linien 13 an der Papierbahn 2, die unter den einzelnen Messmarken 12 der Bahn entlang verlaufen.

In das Prozeß-Leitsystem 7 ist ein Traversierungsablaufprogramm 11 eingesetzt, nach dem der Antriebsmotor 6 gesteuert wird. Das erfolgt so, daß die Traversierungsgeschwindigkeit des Messkopfes 5 nach jeder Überquerung der Papierbahn 3 geändert wird, oder die Dauer der Wartezeit, die zwischen zwei Traversierungen liegt, geändert wird. Es ist auch möglich, sowohl die Traversierungsgeschwindigkeit, als auch die Dauer der Wartezeit ständig zu ändern. Das Ziel des Messverfahrens ist, die Messung entlang jeder der einzelnen Linien 13 in zueinander ungleichen, unregelmäßigen Zeitabständen vorzunehmen.

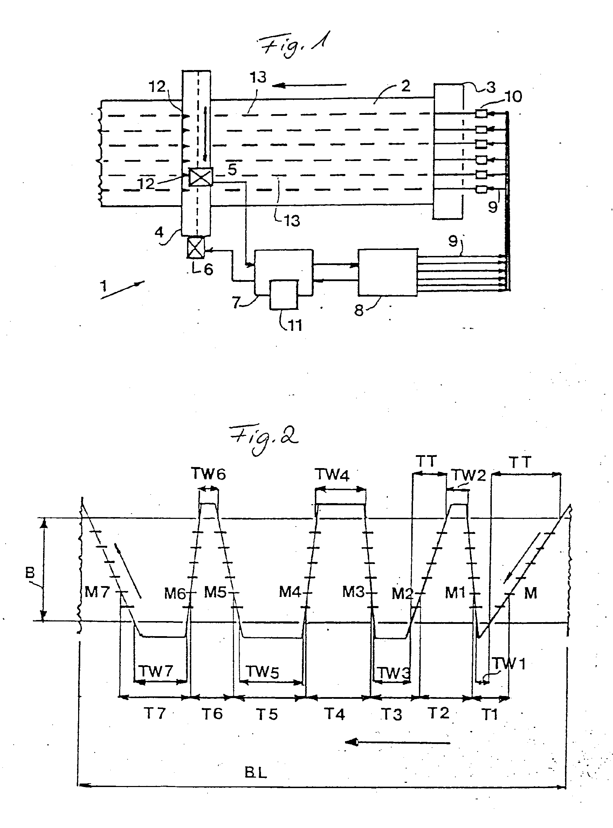

Ein Beispiel solchen Vorgehens ist in Fig. 2 schematisch dargestellt:

Die Folge der Messungen ist: auf einen Längsabschnitt BL der Papierbahn von Breite B gezeigt. Die Bahn bewegt sich in Richtung des zugezeichneten Pfeiles. Die vollgezogene Linie über der Bahn stellt die mit unterschiedlicher Traversierungsgeschwindigkeit erfolgende Bewegung des Messkopfes 5 relativ zur Papierbahn dar. Die Traversierzeit des Messkopfes 5 ist bei den ersten zwei Passagen mit TT bezeichnet. Die einzelnen Messpunkte entlang einer der Linien 13 sind mit M bis M 7 bezeichnet und mit einem Strich in Längsrichtung dargestellt. Der Abstand zwischen den einzelnen Traversierungslinien ist durch die Dauer der jeweiligen Wartezeit TW1 bis TW7 gegeben und bedeutet die Zeit, die zwischen zwei nacheinanderfolgenden Traversierungen liegt, bzw. vorgesehen war. Die Abstände zwischen zwei aufeinanderfolgenden Messungen M bis M7 sind mit T1 bis T7 dargestellt und voneinander verschieden, so daß also die Messungen mit einer sich ändernden Messfrequenz vorgenommen worden sind. Da diese Frequenz ganz willkürlich vorbestimmt und verändert wurde, besteht praktisch keine Wahrscheinlichkeit, daß diese Frequenz mit der Frequenz einer Störung in der Bahnbildung übereinstimmen könnte, was zu einer Verfälschung, bzw. Mißdeutung der Messung führen würde. Die Möglichkeit einer Vortäuschung einer stabilen Querprofilabweichung ist durch die Unregelmäßigkeit der Messung eliminiert.

Das Nachregeln des Öffnungsspaltes des Stoffauflaufes 3 nach statistischer Auswertung der Messungen kann mit Vorteil mittels eines Regelungs-Rechners 8 vorgenommen werden. Seine Steuerbefehle werden über Leitungen 9 den Stellorganen 10 zugeführt, die zum Stellen der lichten Weite des Stoffauflaufspaltes quer zur Bahn 2 vorgesehen sind. Es ist vorstellbar, daß die Steuerbefehle des Regelungs-Rechners 8 auch anderen Stellvorrichtungen zur Beeinflussung des Querprofils der Papierbahn zugeführt werden. Es könnten Stelleinrichtungen sowohl vor als auch nach der Bahnbildungszone der Papiermaschine angesteuert werden.

**Patentansprüche**

1. Messvorrichtung (1) zur Querprofilmessung einer sich hinter einem Stoffauflauf (3) bildenden Papierbahn mit einer ortsfest über der Papierbahn (2) und quer zu ihr angeordneten Messbrücke (4) mit einem an der Messbrücke hin und her, quer zur Papierbahn (2) bewegbaren Messkopf (5) zum Ermitteln des Messwertes (M) zur Beurteilung der Dicke der Papierbahn an dem Messort, über dem sich der Messkopf (5) gerade beim Traversieren auf der Messbrücke entlang der unter ihr laufenden Papierbahn (2) befindet, wobei das Ermitteln der Messwerte jeweils an entlang der Messbrücke vorgesehenen Messorten (12) erfolgt und mit einem Prozeß-Leitsystem (7), das mittels eines Traversierungsablaufprogrammes (11) die Traversierzeit ändern kann, **dadurch gekennzeichnet**, daß das Prozeß-Leitsystem (7) mittels des Traversierungsablaufprogramms (11) die Traversierzeit (TT) und/oder die zwischen zwei Traversierungen liegende Wartezeit (TW) des Messkopfes (5) nach jeder Überquerung der Messbrücke (4) so verändern kann, daß das Ermitteln der Messwerte (M) jeweils an einzelnen Messorten (12) entlang der ablaufenden Papierbahn nach sich ständig ändernden Zeitabständen (T) vorgenommen wird, um durch die sich ändernde Messfrequenz eine Verfälschung bzw. Mißdeutung der Messung infolge einer Störung in der Bahnbildung, deren Frequenz in einem ganzzahligen Verhältnis zu der Meßfrequenz steht, zu vermeiden.

2. Messvorrichtung nach Anspruch 1, dadurch gekennzeichnet, daß sie einen Regelungs-Rechner (8) aufweist zur Ermittelung des stabilen Querprofils über die betrachtete Länge (BL) der Papierbahn (2) aus den Messwerten (M) und zum Regeln der Stellvorrichtungen (10), die das Querprofil der Papierbahn beeinflussen.

3. Anwendung der Messvorrichtung nach Anspruch 2, dadurch gekennzeichnet, daß die Stellvorrichtungen (10) zum Einstellen der lichten Weite des Öffnungsspaltes des Stoffauflaufs (3) regelbar sind.

**Claims**

1. A measuring device (1) for measuring the transverse profile of a paper web forming after a headbox (3), with a measuring bridge (4) located in stationary manner over the paper web (2) and transversely thereto, with a measuring head (5) which can be moved back and forth on the measuring bridge transversely to the paper web (2) for determining the measured value (M) for judging the thickness of the paper web at the measuring point above which the measuring head (5) is located when traversing on the measuring bridge along the paper web (2) which runs beneath it, the determination of the measured values taking place in each case at measuring points (12) provided along the measuring bridge and by means of a process control system (7) which can change the traversing time by means of a traversing sequence program (11), **characterised in that** the process control system (7) can change the traversing time (TT), and/or the waiting time (TW) between two traverses, of the measuring head (5) after each crossing of the measuring bridge (4) by means of the traversing sequence program (11) such that the determination of the measured values (M) is performed in each case at individual measuring points (12) along the passing paper web in constantly changing intervals of time (T), in order to avoid falsification or misinterpretation of the measurement as the result of a disruption in the formation of the web, the frequency of which is in an integer relationship to the measuring frequency, due to the changing measuring frequency.

2. A measuring device according to Claim 1, characterised in that it has a control computer (8) for determining the stable transverse profile over the length (BL) of the paper web (2) being looked at from the measured values (M) and for controlling the setting devices (10) which affect the transverse profile of the paper web.

3. Application of the measuring device according to Claim 2, characterised in that the setting devices (10) are controllable for setting the internal width of the opening gap of the headbox (3).

**Revendications**

1. Dispositif de mesure (1) pour mesurer le profil transversal d'une bande de papier se formant à la sortie d'une caisse de tête (3) avec un pont de mesure (4) disposé à poste fixe au-dessus de la bande de papier (2) et transversalement à celle-ci, avec une tête de mesure (5) mobile en va-et-vient sur le pont de mesure, transversalement à la bande de papier (2), pour déterminer la valeur de mesure (M) pour l'évaluation de l'épaisseur de la bande de papier à l'emplacement de mesure au-dessus duquel la tête de mesure (5) se trouve précisément lors de son mouvement transversal sur le pont de mesure le long de la bande de papier (2) circulant sous ladite tête de mesure, tandis que la détermination des valeurs de mesure a lieu chaque fois en des emplacements de mesure (12) prévus le long du pont de mesure, et avec un système de gestion de process (7) qui peut modifier, au moyen d'un programme (11) de déroulement du mouvement transversal, la durée de ce mouvement,

   **caractérisé** en ce que le système de gestion de process (7) peut, au moyen du programme (11) de déroulement du mouvement transversal, modifier la durée (TT) de ce mouvement et/ou la durée du temps d'attente (TW) de la tête de mesure (5) entre deux traversées, après chaque parcours transversal sur le pont de mesure (4), de telle sorte que la détermination des valeurs de mesure (M) a lieu chaque fois en des emplacements de mesure (12) individuels le long de la bande de papier en circulation, à des intervalles de temps (T) qui changent constamment, afin d'éviter, grâce à ce changement continu de la fréquence de mesure, une distorsion ou une fausse interprétation de la mesure par suite d'une perturbation dans la formation de la bande de papier, dont la fréquence se trouve dans un rapport s'exprimant par un nombre entier par rapport à la fréquence de mesure.

2. Dispositif de mesure selon la revendication 1, caractérisé en ce qu'il présente un calculateur de régulation (8) pour la détermination du profil transversal stable sur la longueur considérée (BL) de la bande de papier (2) d'après le valeurs de mesure (M) et pour la régulation des dispositifs de réglage (10) qui influencent le profil transversal de la bande de papier.

3. Utilisation du dispositif de mesure selon la revendication 2, caractérisée en ce que les dispositifs de réglage (10) pour le réglage de l'ouverture de la fente de sortie de la caisse de tête (3) peuvent être régulés.

Fig. 1

Fig. 2